# EUROPÄISCHE PATENTANMELDUNG

(11) **EP 0 931 766 A2**
(43) Veröffentlichungstag der Anmeldung: **28.07.1999**
(21) Anmeldenummer: 99250023.1
(22) Anmeldetag: 21.01.1999
(51) Int. Cl.: C02F 1/48, C02F 1/44, C02F 1/32, G05D 21/02, G01N 27/26

(54) **Verfahren und Anlage zur Wasseraufbereitung, insbesondere für Wasserstrahlschneidanlagen**

(30) Priorität: 23.01.1998 DE 19803967
(71) Anmelder: Intrec Gesellschaft für Innovative Technologien mbH, 12526 Berlin (DE)
(72) Erfinder: Weide, Hans-Günter, Dr., 12623 Berlin (DE)
(74) Vertreter: Hengelhaupt, Jürgen, Dipl.-Ing.

(57) **Zusammenfassung**

Die Erfindung bezieht sich auf ein Verfahren und eine Anlage zur Wasseraufbereitung, insbesondere für Wasserstrahlschneidanlagen, unter Verwendung eines Betriebssteuerrechners, wobei in einer Eingangswasseraufbereitungs- und Enthärtungsanlage dem Wasser die gelösten Calcium- und Magnesiumionen entzogen werden und in einer weiteren Anlage die im Wasser gelösten Schwermetallionen, die biologischen Bestandteile des Wassers und die Feinstpartikel reduziert werden.

Die Aufgabe der Erfindung, ein Verfahren und eine Anlage zur Wasseraufbereitung für Wasserstrahlschneidanlagen zu entwickeln, mit denen die von den Herstellern von Wasserstrahlhochdruckpumpen geforderten individuellen Parameter der Wasserqualität und mit denen eine höhere Standzeit der Pumpen als bisher bekannt gewährleistet werden und mit denen ein geschlossener Kreislaufprozeß erreicht wird, wird dadurch gelöst, daß in einem geschlossenen Kreislaufprozeß die Parameter Wasserhärte, Leitfähigkeit, Radikalen- und Schwermetallionenbelastung über rechnergesteuerte spezifische Anlagenkomponenten programmierbar eingestellt und ständig kontrolliert und geregelt werden.

## Beschreibung

Die Erfindung betrifft ein Verfahren und eine Anlage zur Wasseraufbereitung, insbesondere für Wasserstrahlschneidanlagen, gemäß den Oberbegriffen der Ahsprüche 1 und 6.

Insbesondere Hochdruckwasserstrahlschneidanlagen benötigen zu ihrer effektiven Arbeit Qualitätswasser mit definierten Parametern. Ein geschlossener Wasser-Kreislauf kann dabei Kosten- und Umweltbelastung reduzieren.

Das Wasser muß, als Träger des technologischen Schneidprozesses beim Wasserstrahlhochdruckschneiden, so aufbereitet werden, daß die gesamten im Wasser gelösten Ionen die angestrebten Eigenschaften des Wassers ergeben.

Neben der Wasserhärte sind die Parameter Leitfähigkeit, die Radikalen- und Schwermetallionenbelastungen zu beeinflussen.

Die Wasserhärte wird von der im Wasser gelösten Calcium- und Magnesiumionenanzahl bestimmt. Diese und die weiteren im Wasser gelösten Ionen bestimmen dessen Leitfähigkeit.

Untersuchungen haben ergeben, daß das Wasser eine kristallähnliche Nahordnung besitzt. Wird diese Ordnung durch starke mechanische Einflüsse, wie z.B. durch das Wasserstrahlschneiden, zerstört, dann können sogenannte Radikale entstehen, die als freie Sauerstoffbindungen eine hohe Aggressivität gegenüber mit diesem Wasser in Berührung kommenden Materialien erzeugen.

Für eine lange Lebensdauer und Funktionsfähigkeit der Wasserhochdruckpumpen in den Wasserstrahlschneidanlagen, die mit Drücken zwischen 3000 und 5000 bar arbeiten, sind für die unterschiedlichen Wasserstrahlhochdruckpumpen unterschiedliche Parameter für die Wasserqualität zu gewährleisten.

Bekannte Wasseraufbereitungsverfahren und Anlagen behandeln das Wasser im wesentlichen hinsichtlich der Härte durch Anlagen zur Kalkausscheidung der gelösten Calcium- und Magnesium-Ionen.

Aufgabe der Erfindung ist es, ein Verfahren und eine Anlage zur Wasseraufbereitung für Wasserstrahlschneidanlagen zu entwickeln, mit denen die von den Herstellern von Wasserstrahlhochdruckpumpen geforderten individuellen Parameter der Wasserqualität gewährleistet werden können und mit denen eine höhere Standzeit der Pumpen als bisher bekannt realisiert werden kann sowie ein geschlossener Wasserkreislaufprozeß ermöglicht wird.

Diese Aufgabe wird durch die Merkmale der Ansprüche 1 und 6 gelöst. Zweckmäßige Ausgestaltungen der Erfindung sind in den Unteransprüchen enthalten. Die besonderen Vorteile der beanspruchten technischen Lehre bestehen darin, daß durch die gezielte Kontrolle und Regelung der die Wasserqualität beeinflussenden Parameter, insbesondere der Leitfähigkeit des Wassers und der Menge der freien Radikalen, eine den eingesetzten Pumpen adäquate Wasserqualität bereitgestellt werden kann, die zu einer wesentlichen Erhöhung bzw. Angleichung der Standzeiten bei den kostenintensiven Wasserstrahlhochdruckpumpen und allen wasserführenden Bauteilen der Wasserstrahlschneidanlage führen. Die Parameter Wasserhärte, Leitfähigkeit, Radikalen- und Schwermetallionenbelastung wird über rechnergesteuerte spezifische Anlagenkomponenten programmierbar eingestellt. Durch die ermöglichte Wiederverwendung des Wassers im Kreislaufprozeß und durch den dadurch erreichten geschlossenen Kreislaufprozeß wird eine wesentlich geringere Umweltbelastung als nach dem bekannten Stand der Technik gewährleistet. Der Kreislaufbetrieb des Wassers ist über einen Zeitraum bis zur Grundreinigung der Wasserstrahlschneidanlage möglich. Der Wasserverbrauch ist erheblich gesenkt und beschränkt sich bei der wiederholten Aufbereitung auf die Ergänzung von verspritztem und verdunstetem Wasser sowie auf die geringen Mengen zur Rückspülung (< 10%). Die Wasseraufbereitungsanlage ist in der Lage, nach einer groben Wasseranalyse des Speisewassers im Netz, selbständig die benötigte Qualität des Schneidwassers herzustellen.

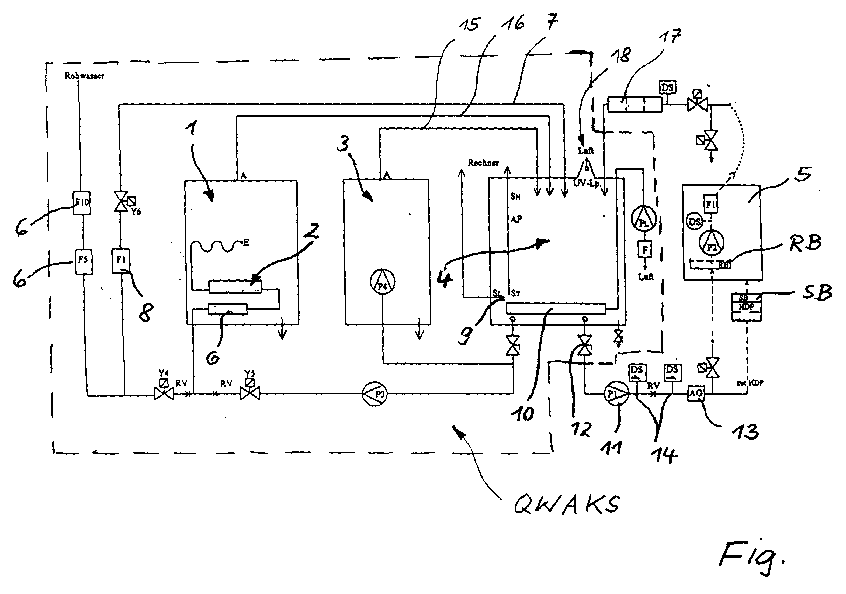

Die Erfindung soll nachstehend anhand eines in der einzigen Figur dargestellten Ausführungsbeispiels eines Wasseraufbereitungs- und Kreislaufsystems für das Wasserstrahlschneiden näher erläutert werden. Die einzige Figur zeigt in einer schematischen Darstellung das Verfahrensfließbild des Wasserkreislaufsystems nach der Erfindung.

Die als Qualitätswasseraufbereitungs- und Kreislaufsystem (QWAKS) bezeichnete Wasseraufbereitungsanlage besteht im wesentlichen aus den Anlagenkomponenten,
- Eingangswasseraufbereitungs- und Enthärtungsanlage 1, zum Beispiel einer Revers Osmose-Anlage, die mit einer Anlage 2 zur Kalkausscheidung der gelösten CalciumIonen mittels Magneten, nachfolgend Magnetoflex genannt, verbunden ist, - einer Anlage 3 zur Reduzierung der Schwermetallionen, aller biologischen Bestandteile des Wassers und der Feinstpartikel sowie der Radikalen Verminderung,
- einem Wassertank 4, in welchem das aufbereitete Schneidwasser in der für die jeweiligen Hochdruckpumpentypen geforderten Wasserqualität enthalten bzw. gespeichert ist.
Die Anlage QWAKS arbeitet mit
- einer Recyclinganlage 5, in der eine Vorsäuberung bzw. Wiederaufbereitung des Wassers zur Wiederverwendung im Kreislaufsystem erfolgt, in der der Feststoffgehalt des Wassers bis auf Teile <1µm reduziert wird und in der das wiederverwendbare Abrasiv aus der Schneidanlage zurückgewonnen wird, und mit
- einem Betriebssteuerrechner, der alle Anlagenkomponenten automatisch steuert (nicht dargestellt), zusammen.

Beim Wasserstrahlschneiden wird das Wasser mit Abrasivsand und mit Anteilen der geschnittenen Stoffe verunreinigt. Der größte Teil ist als schwebender Feststoff im Wasser enthalten, der von mikroskopisch kleinen Größen bis zu einem Durchmesser von knapp einem Millimeter reicht. Weiterhin werden Ionen im Wasser gelöst, die auch - wie bei Eisen - Komplexe bilden können, von denen Teile löslich als auch unlöslich sind.

Wird das Wasser nur einmal benutzt, liegt der Anteil der gelösten Schwermetallionen und anderer störenden Ausfällung unterhalb des für die Einleitung des Wassers in die Kanalisation geforderten Nachweisbereiches. Bei wiederholtem Einsatz des Wassers würde diese Konzentration jedoch den Nachweisbereich überschreiten und schädliche Einflüsse auf die wasserführenden Bauteile der Wasserstrahlschneidanlage, insbesondere der Hochdruckpumpe, nehmen.

Aus diesen Gründen sind für die Aufbereitung des Wassers zur Wiederverwendung im Kreislaufsystem mehrere Stufen vorgesehen. Zum Ersten wird über die Recyclinganlage 5 der Feststoffgehalt des Wassers drastisch bis auf Teile <1µm reduziert. Dabei werden mehr als 50% des wiederverwendbaren Abrasivs zurückgewonnen. Das von der Recyclinganlage 5 abgegebene einleitungsfähige Abwasser wird nun von der Anlage QWAKS aufgenommen, hochfein gefiltert und von den mit fortschreitendem Kreislaufprozeß wachsenden Anteilen von Ionen und Wasserhärte befreit.

In offenen Wassersystemen, die längere Zeit (mehr als ein Monat) im Kreislauf betrieben werden, wachsen Bakterien, Pilze und Algen als Verunreinigung im Wasser. Diese biologischen Bestandteile führen schnell zum Faulen des Wassers, was mit unangenehmen Gerüchen, Gasentwicklung und Verstopfungen von Gefäßen und Ventilen verbunden ist.

Die Wasseraufbereitungsanlage QWAKS verhindert in seinen Anlagekomponenten auch das Vermehren und Anwachsen der biologischen Komponenten, so daß ein Kreislaufbetrieb des Wassers über den Zeitraum bis zur Grundreinigung der Wasserstrahlschneidanlage (1/2 Jahr) möglich ist. Wasserverluste, die durch Verspritzen oder Verdunstung im System entstehen, werden durch Zuleitung aus dem Wassernetz ergänzt. Der zusätzliche Wasserverbrauch im Kreislaufsystem liegt weit unter 10%.

Die Anlage arbeitet auf der Basis der Erkenntnis, daß einmal optimal eingestellte Wasserqualitäten, die in den Kreislaufprozeß des Wasserstrahlschneidens eingeführt werden, durch den Wasserstrahlschneidprozeß nur langsam in negative Richtung verändert werden.

Die Betriebsphilosophie der Anlage QWAKS ist deshalb darauf ausgerichtet, daß das gesamte System, bestehend aus Wassertank 4, Reservebecken (RB) der Recyclinganlage 5 und Schneidbecken (SB) der Wasserstrahlschneidanlage, durch aufbereitetes Wasser aus der Anlage QWAKS gefüllt wird. Dadurch wird gewährleistet, daß im gesamten System eine der Wasserstrahlpumpe optimal angepaßte Wasserqualität als Ausgangsfüllung vorhanden ist.

Zur Gewinnung der Eingangswasserqualität wird das zur Verfügung stehende Rohwasser aus dem Stadtwasser oder vorbereitetes Wasser über Filter 6 dem Entkalkungssystem 2 zugeführt. Vorzugsweise wird dafür das System Magnetoflex benutzt, das den im Wasser gelösten Kalk durch Keimbildung in so großen Kalkkonzentrationen bindet, daß sie in der nachfolgenden Anlage 1 (Revers Osmose) keine Filterporen verstopft und mit dem Konzentrat ausgeschieden werden. Der Vorteil des Einsatzes des Magnetoflex-Systems 2 liegt darin, daß keine Natriumionenbeaufschlagung erfolgt, die unausweichlich beim Einsatz eines Ionenaustauschers üblicher Art wären. In der nachgeschalteten klassischen Anlage 1 der Revers Osmose wird eine extreme Reinigung des Wassers erreicht, was sich durch eine Verringerung der Leitfähigkeit auf bis zu 20µS/cm ausdrückt. Dieses Wasser wird in den Wassertank 4 der Anlage QWAKS als Reservebehälter gegeben. Von den jeweiligen Herstellern der Wasserstrahlhochdruckpumpen werden unterschiedliche Wasserqualitäten gefordert. Eine typische Wasserqualitätsanforderung verlangt einen Mindestgesamtionenanteil im Wasser von einer Leitfähigkeit von 110 µS/cm. Reines Revers Osmose-Wasser mit 20 µS/cm gilt als aggressiv und wird von den meisten Wasserstrahlhochdruckpumpen-Herstellern verworfen. Um die genannte Forderung zu erfüllen, wird über einen zweiten rechnergesteuerten Zulauf 7 Verschneidwasser in den Wassertank 4 gegeben. Dieses Verschneidwasser wird aus dem Rohwasser abgeleitet und mit einem zusätzlichen Feinfilter 8 als Verschneidwasser so lange in den Wassertank 4 gegeben, bis die Solleitfähigkeit im Wassertank 4 der Anlage QWAKS erreicht wird.

Dabei wird die Leitfähigkeit im Wassertank 4 kontinuierlich über eine Leitfähigkeitssonde 9 gemessen und einem nicht dargestellten Steuerrechner zugeführt, der die Menge des Verschneid- und des Rohwassers über die Osmose in der Anlage 1 steuert. Im Wassertank 4 wird über einen Belüfter (Perlator 10) durch Belüftung für eine effektive Durchmischung des Wassers gesorgt, so daß die Leitfähigkeitsmessung einen repräsentativen Wert für den Gesamtbehälter 4 ergibt, obwohl sie nur an einer Stelle des Tank 4 abgenommen wird. Mit diesem Ausgangswasser im Wassertank 4, welches den optimalen Anforderungen der Wasserstrahlhochdruckpumpen-Hersteller entspricht, und welches durch die Rechnersteuerung an die jeweiligen Forderungen der Pumpenhersteller angepaßt werden kann, wird das Gesamtsystem, das aus Wasserstrahlschneidemaschine und Abrasiv-Recyclinganlage 5 besteht, zugeführt. Dabei wird zuerst das Reservewasserbecken (RB) der Recyclinganlage 5 mit dem Systemwasser aus dem Wassertank 4 der Anlage QWAKS aufgefüllt und anschließend über die Steuerung der Anlage 5 das Schneidbecken (SB) der Wasserstrahlschneidemaschine gefüllt, das zum Abbremsen des Wasserstrahles und Auffangen der Abrasiv- und Schneidmittel in der Regel als statisches großformatiges Becken ausgeführt ist. Mit diesem Vorgehen wird erreicht, daß im gesamten System, das anschließend im Arbeitszyklus als Kreislauf geschaltet wird (entsprechend dem vorliegenden Schema), mit Qualitätswasser gefüllt wird, das den Anforderungen der Wasserstrahlhochdruckpumpen-Hersteller entspricht.

Im Betriebsfall, der nach vollständigem Füllen des Systems bis zu den Arbeitswasserniveaus des Reserve- und Schneidbeckens erreicht wird, wird rechnergesteuert das Ventil 12 zum Füllen geschlossen und mit der Pumpe 11 (P1) der Arbeitsdruck zur Hochdruckpumpe HDP hergestellt. Über ein Ausgleichsgefäß 13 und einen Druckwächter 14 wird verhindert, daß die Pumpe 11 (P1) gegen starken Druck läuft, wenn die Hochdruckpumpe HDP keinen Wasserverbrauch anzeigt. Mit dem Schneidprozeß gelangen pro Schneidkopf ca. 200 l Wasser pro Stunde in das Schneidbecken SB, was zum kontinuierlichen Niveauanstieg des Schneidbeckens SB führen würde. Von der Recyclinganlage 5 wird jedoch der Abrasivsand mit den Schneidresten kontinuierlich über ein Pumpensystem aufgesaugt und gesäubertes Wasser so an das Schneidbecken SB zurückgegeben, daß dessen Niveau entsprechend der eingestellten Arbeitshöhe konstant bleibt.

Das durch den Schneidprozeß zusätzlich in das Schneidbecken SB der Anlage 5 gelangte Wasser befindet sich nun im Reservebecken RB der Anlage 5 und wird ebenfalls rechnergesteuert über einen Ausgangsfilter 17 an die Anlage QWAKS zurückgegeben, so daß im Arbeitszyklus kein Rohwasser mehr verbraucht wird. Es werden dann nur noch Wasserverluste aus Verdunsten, Verspritzen und Spülzyklen ersetzt. Der Wasserverbrauch liegt dabei wesentlich unter 10 % der Gesamtfüllung. Bei der Übergabe des Wassers erfolgt in der Anlage QWAKS eine Ultrafeinfilterung, so daß alle störenden Feststoffanteile zurückgehalten werden. Durch die Erkenntnis, daß während des Schneidprozesses die Wasserqualitätsparameter bis auf die Verunreinigung durch Feststoffe im wesentlichen erhalten bleiben, liegen im Wassertank 4 nach dem Reinigungsprozeß wieder die geforderten Qualitätsparameter des Wassers vor.

Beim Wasserstrahlschneidprozeß wird die Nahordnung der Wassermoleküle zerschlagen und es entstehen Radikale, die mehrere Monate Lebensdauer besitzen und zur ständig steigenden Aggressivität des Wassers beitragen.

Durch eine ständige kräftige Durchlüftung des Wassers, durch den im Wassertank 4 angebrachten Perlator 10, wird als erstes ein Faulen und übermäßiges Vermehren von biologischen Anteilen im Wassertank 4 durch kräftige Durchmischung mit Sauerstoff verhindert. Gleichzeitig fallen dabei die gefürchteten Eisen- und anderen Komplexsalze weitgehend aus, die sonst erst in der Druckphase der Wasserstrahlhochdruckpumpe HDP Ðausfallen und zu Beschädigungen der empfindlichen Oberflächen der bewegten Teile führen würden.

Durch eine harte UV-Belichtung 18 des sprudelnden Wassers an der Oberfläche wird ein Abtöten der Bakterien erreicht und eine Reduzierung der beim Schneidprozeß entstandenen Radikalen verursacht. Die Belüftung des Wassertanks 4 über den Perlator 10 und die Bestrahlung mit hartem UV-Licht 18 wird durch ein Standby-System auch bei Betriebspausen des Wasserstrahlschneidprozesses kontinuierlich gesichert, so daß auch längere Pausen - wie Wochenende, Werksferien - in denen Wasser im Wassertank 4 bleibt, keine ungewollte wesentliche Verschlechterung der Wasserqualität im Wassertank 4 eintritt.

Diese Maßnahmen reichen jedoch nicht aus, um die Wasserqualität stabil zu halten, wenn kontinuierlich geschnitten wird. Aus diesem Grund ist zum Wassertank 4 ein Bypass 15 angeschlossen, in dem die Anlage 3 eingeschlossen ist. Die Anlage 3 besteht aus zwei Komponenten. Zum einen aus einem Elektrosorbtionssystem, was gestattet, die entstehenden Schwermetallionen und Feinstpartikel dem Wasser zu entziehen. Gleichzeitig werden alle biologischen Anteile abgetötet und somit unwirksam gemacht. Mit einem Katalysator wird als zweite Funktion eine effektive Reduzierung der Radikalen erreicht und so das Wasser in die geforderte Qualität für die Hochdruckpumpe HDP gebracht. Dieses Wasser wird in den Wassertank 4 zurückgegeben. Im Wassertank 4 steht nun wieder hochqualitatives Wasser für die Hochdruckpumpe HDP zur Verfügung. Da der Schneidprozeß produktionsbedingt auch Pausen beinhaltet, ist der Bypass 15 über die Anlage 3, die ständig während der Betriebsphasen arbeitet, mit den nötigen Reserven versehen, so daß alle entstehenden Nebenprodukte in ausreichender Menge beseitigt werden können.

Für den Fall, daß im Wasserstrahlschneidprozeß kalkhaltige Materialien oder Materialien geschnitten werden, die in anderer Weise die Wasserhärte erhöhen, wird dieser Zustand durch die ständige Kontrolle der Leitfähigkeit im Wassertank 4 der Anlage QWAKS angezeigt. Übersteigt die Leitfähigkeit ein durch den Rechner kontrolliertes Maß, wird der Bypass 15 zur Anlage 3 abgeschaltet und ein zweiter Bypass 16 über die Anlage 2 zur Entkalkung (Magnetoflex) und die Anlage 1 (Revers Osmose) geschaltet. Dieser Bypass 16 reduziert effektiv die Ionenkonzentration, die zur Erhöhung des Leitwertes und zur Qualitätsverschlechterung des Wassers bezüglich der Anforderung der Wasserstrahlhochdruckpumpe HDP führen. Ist nach relativ kurzer Zeit wieder der Sollwert der Leitfähigkeit im Wassertank 4 der Anlage QWAKS erreicht, wird, durch den Rechner gesteuert, dieser Bypass 16 wieder auf den Bypass 15 zu Anlage 5 umgeschaltet, der wiederum - wie beschrieben - die schädlichen Konzentrationen von Schwermetallionen, biologischen und Feinstfeststoffkomponenten reduziert sowie die Radikalenbildung rückgängig macht.

Die Erfindung ist nicht beschränkt auf die hier dargestellten Ausführungsbeispiele. Vielmehr ist es möglich, durch Kombination und Modifikation der genannten Mittel und Merkmale weitere Ausführungsvarianten zu realisieren, ohne den Rahmen der Erfindung zu verlassen.

### Bezugszeichenliste

- 1: Eingangswasseraufbereitungs- und Enthärtungsanlage
- 2: Magnetoflex-System
- 3: Anlage zur Reduzierung der Schwermetallionen usw.
- 4: Wassertank
- 5: Recyclinganlage
- 6: Filter
- 7: Zulauf Verschneidwasser
- 8: Feinfilter
- 9: Leitfähigkeitssonde
- 10: Perlator
- 11: Pumpe P1
- 12: Ventil
- 13: Ausgleichsgefäß
- 14: Druckwächter
- 15: Bypass
- 16: Bypass
- 17: Filter
- 18: UV-Belichtung
- A,E: Eingang, Ausgang
- DL: Druckluft
- DS: Druckschalter
- F: Filter
- P: Pumpe
- RV: Rückschlagventil
- SH,ST: Niveausensoren
- Y: Magnetventil
- HDP: Hochdruckpumpe
- RB: Reservebecken
- SB: Schneidbecken

## Patentansprüche

1. Verfahren zur Wasseraufbereitung, insbesondere für Wasserstrahlschneidanlagen, wobei in einer Eingangswasseraufbereitungs- und Enthärtungsanlage dem Wasser die gelösten Calcium- und Magnesiumionen entzogen werden und/oder in einer weiteren Anlage die im Wasser gelösten Schwermetallionen, die biologischen Bestandteile des Wassers und die Feinstpartikel reduziert werden,
dadurch gekennzeichnet, daß
in einem geschlossenen Kreislaufprozeß die Parameter Wasserhärte und/oder Leitfähigkeit und/oder Radikalen- und/oder Schwermetallionenbelastung über rechnergesteuerte spezifische Anlagenkomponenten programmierbar eingestellt und ständig kontrolliert und geregelt werden.

2. Verfahren nach Anspruch 1
dadurch gekennzeichnet, daß
die Leitfähigkeit des Wassers in einem Wassertank (4) integral gemessen und durch den Rechner ausgewertet und im Ergebnis entsprechende Anlagenkomponenten im Bypassbetrieb an- und abgeschaltet werden.

3. Verfahren nach Anspruch 2,
dadurch gekennzeichnet, daß
über einen rechnergesteuerten Zulauf (7) Verschneidwasser bzw. zusätzlich feingefiltertes Rohwasser bis zur Erreichung der Soll-Leitfähigkeit in den Wassertank (4) gegeben wird, wobei die Leitfähigkeit im Wassertank (4) über eine Leitfähigkeitssonde (9) gemessen und dem Steuerrechner zugeführt wird.

4. Verfahren nach Anspruch 1,
dadurch gekennzeichnet, daß das Wasser im Wassertank (4) mit Sauerstoff durchmischt wird, um Faulen und übermäßigen Vermehren von biologischen Anteilen zu verhindern und um eine Ausfällung von Eisen- und anderen Komplexsalzen zu erreichen, daß die Oberfläche des im Wassertank (4) sprudelnden Wassers einer harten UV-Belichtung (18) ausgesetzt wird, um ein Abtöten von Bakterien und eine Reduzierung der beim Schneidprozeß entstandenen Radikalen zu erreichen, daß die beim kontinuierlichen Schneiden anfallenden Schwermetallionen und Feinstpartikel dem Wasser entzogen werden.

5. Verfahren nach den Ansprüchen 1 bis 4,
dadurch gekennzeichnet, daß
das aus dem Wasserstrahlschneidesystem entnommene Wasser zur Wiederverwendung wiederaufbereitet wird, indem
- über die Recyclinganlage (5) der Feststoffgehalt des Wassers bis auf Teile <1 µm reduziert wird,
- das von der Recyclinganlage (5) abgegebene, einleitungsfähige Abwasser hochfein gefiltert und
- von der mit fortschreitenden Kreislaufprozeß wachsenden Anteilen von Ionen und Wasserhärte befreit wird.

6. Anlage zur Wasseraufbereitung für Wasserstrahlschneidanlagen, insbesondere in Verbindung mit mindestens einer Hochdruckpumpe HDP der Wasserstrahlschneidmaschine und einem Recyclingsystem (5), zur Steuerung aller Anlagenkomponenten,
dadurch gekennzeichnet, daß
ein Wassertank (4) mit
- einer Eingangswasseraufbereitungs- und Enthärtungsanlage (1) mit einem System (2) zur Kalkausscheidung der gelösten Calcium-Ionen und/oder mit
- einer Anlage (3) zur Reduzierung der Schwermetallionen und/oder aller biologischen Bestandteile des Wassers und/oder der Feinstpartikel und/oder der Verminderung der Radikalen und/oder mit
- der Recyclinganlage (5) zu einem Kreislaufsystem zusammengeschlossen sind.

7. Anlage nach Anspruch 6,
dadurch gekennzeichnet, daß
- eine Rohwasserleitung über Filter (6) Magnetventile (4) und Rückschlagventile (RV) mit der Eingangswasseraufbereitungs- und Enthärtungsanlage (1) und mit dem Wassertank (4) verbunden ist,
- über einen Leitfähigkeitssensor (9) und über den Betriebssteuerrechner eine Verschneidewasserleitung (7) mit dem Filter (F1) und dem Steverventil (I6) mit dem Wassertank (4) verbunden ist,
- der Wassertank (4) über eine Leitung, in der eine Pumpe (P1) und ein Schaltventil (Y1) angeordnet sind, mit einem Reservebecken (RB) verbunden ist,
- das Reservebecken (RB) über eine Leitung, in der eine Rückführpumpe angeordnet ist, mit einem Schneidbecken (SB) der Hochdruckwasserschneid-Anlage verbunden ist,
- ein Ausgleichsgefäß (AG) zwischen dem Wassertank (4) und der Hochdruckpumpe (HDP) geschaltet ist.

8. Anlage nach den Ansprüchen 6 und 7,
dadurch gekennzeichnet, daß
die Betriebssteuerrechner der Wasseraufbereitungsanlagen (QWAKS) und der Recyclinganlage (5) über Busleitungen so vernetzt sind, daß die Funktionen der einzelnen Anlagenkomponenten (4, 1, 2, 3, 5, 10) einschließlich der eingefügten Bypässe (15, 16) koordiniert gesteuert werden.

9. Anlage nach Anspruch 6,
dadurch gekennzeichnet, daß
der Wassertank (4) eine Luftdurchmischungseinrichtung (10) und eine Niveauregelung (SH, ST) aufweist.
